# EUROPÄISCHE PATENTSCHRIFT

(11) **EP 1 328 259 B1**
(45) Veröffentlichungstag und Bekanntmachung des Hinweises auf die Patenterteilung: **09.04.2014**
(21) Anmeldenummer: 01988570.6
(22) Anmeldetag: 18.10.2001
(51) Int. Cl.: A61K 9/70

(54) **TRANSDERMALE THERAPEUTISCHE SYSTEME MIT LICHTEMPFINDLICHEN WIRKSTOFFEN**
TRANSDERMAL THERAPEUTIC SYSTEMS COMPRISING PHOTOSENSITIVE ACTIVE SUBSTANCES
SYSTÈMES THÉRAPEUTIQUES TRANSDERMIQUES COMPRENANT DES PRINCIPES ACTIFS PHOTOSENSIBLES

(30) Priorität: 27.10.2000 DE 10053375
(43) Veröffentlichungstag der Anmeldung: 23.07.2003
(73) Patentinhaber: LTS LOHMANN Therapie-Systeme AG, 56626 Andernach (DE)
(72) Erfinder: DEGEN, Anja, 56653 Wehr (DE); THEOBALD, Frank, 53498 Bad Breisig (DE)
(74) Vertreter: Bode, Birte
(86) Internationale Anmeldenummer: PCT/EP2001/012068
(87) Internationale Veröffentlichungsnummer: WO 2002/034200

(56) Entgegenhaltungen:
- EP-A2- 1 129 696
- WO-A1-01/66077
- WO-A1-91/09731
- DE-A1- 19 753 983
- DE-A1- 19 912 623
- DATABASE WPI Section Ch, Week 199540 Derwent Publications Ltd., London, GB; Class B02, AN 1995-307213 XP002212205 & JP 07 204251 A (DAIICHI PHARM CO LTD), 8. August 1995 (1995-08-08)
- DATABASE WPI Week 198525, Derwent Publications Ltd., London, GB; AN 1985-131655 & JP 60 069014 A (NITTO ELECTRIC IND. CO.) 19 April 1985

## Beschreibung

Die vorliegende Erfindung betrifft transdermale therapeutische Systeme (TTS), die lichtempfindliche Wirkstoffe enthalten und die farblos und transparent sind.

Verschiedene pharmazeutisch wirksame Substanzen, z. B. Nicotin oder Nifedipin, weisen eine hohe Lichtempfindlichkeit auf. Bei Arzneimittelzubereitungen, die solche lichtempfindliche Wirkstoffe enthalten, kann es unter Einwirkung des Tages- bzw. Sonnenlichts zu einer photochemischen Zersetzung des Wirkstoffs und infolgedessen zu einer signifikanten Verringerung des Wirkstoffgehalts kommen, wenn die Wirksubstanzen während der Lagerung der Zubereitungen bis zum Zeitpunkt der Applikation, oder während der Applikationsdauer, nicht vor Lichtzutritt geschützt werden.

Bei den klassischen Applikationsformen, wie z. B. oralen, parenteralen oder konjunctival zu applizierenden Darreichungsformen, wird eine ausreichende Stabilität gegen Lichteinwirkung meist bereits dadurch erhalten, daß eine geeignete Primär- oder Sekundärverpackung gewählt wird, die den Zutritt von Tageslicht zum Wirkstoff verhindert. Da zwischen der Entnahme der Zubereitung aus der Verpackung und ihrer Verabreichung in der Regel nur ein kurzer Zeitraum liegt, ist eine Zersetzung des Wirkstoffs infolge von Lichteinwirkung bei diesen Arzneiformen weitgehend ausgeschlossen. Falls eine längere Applikationsdauer erforderlich ist, wie z. B. bei der Anwendung von Infusionslösungen, so erfolgt die Applikation meist stationär, wobei eingefärbte oder sekundärverpackte Infusionsflaschen verwendet werden können, um die lichtempfindlichen Wirkstoffe vor Zersetzung zu schützen.

Die genannten Maßnahmen sind in der Regel ausreichend, um die Stabilität des zu applizierenden Wirkstoffs während der Lagerung bzw. während der Applikationsdauer zu gewährleisten.

Von diesen klassischen Applikationsformen unterscheiden sich allerdings die transdermalen therapeutischen Systeme (TTS). Diese stellen mit Wirkstoff beladene Systeme dar, wobei die Wirkstoffe in selbstklebenden oder nichtselbstklebenden Polymeren unterschiedlicher chemischer Zusammensetzung enthalten sind. Die enthaltenen Wirkstoffe werden kontinuierlich über einen längeren Zeitraum an die Haut des Patienten abgegeben, d. h. ein TTS wird auf die Haut appliziert und verbleibt dort für einen längeren Zeitraum, beispielsweise einige Stunden bis mehrere Tage.

Infolgedessen ist der Wirkstoff bei den genannten Applikationsformen (TTS) auch während der Applikationsdauer, abhängig vom jeweiligen Applikationsort, mehr oder weniger stark dem Tageslicht ausgesetzt und kann während seiner Applikationsdauer einen signifikanten, nicht vernachlässigbaren Wirkstoffverlust erfahren. Dies kann im Extremfall, beispielsweise bei besonders lichtempfindlichen Wirksubstanzen, zum Unterschreiten der therapeutisch notwendigen Wirkstoffzufuhr führen und damit den Therapieerfolg gefährden.

Bei auf dem Markt befindlichen TTS, die lichtempfindliche Wirkstoffe enthalten, wird das Problem in der Regel dadurch gelöst, daß eine aluminisierte oder lackierte Abdeckfolie verwendet wird. Diese bildet die Rückschicht des Systems und bedeckt die wirkstoffhaltige Matrix nach außen hin, so daß der Zutritt des Tageslichts zur wirkstoffhaltigen Matrix minimiert wird und dadurch der Wirkstoff vor der Zersetzung durch Sonnenlicht geschützt wird.

Beispielsweise wird in DE-A1-199 12 623 vorgeschlagen, zur Verbesserung der Stabilität lichtempfindlicher TTS diese mit eingefärbten Kunststoffolien als Abdeckfolien auszustatten.

Diese Methode des Lichtschutzes unter Verwendung aluminisierter, lackierter oder eingefärbter Abdeckfolien kann jedoch in einigen Fällen unerwünscht sein oder zu Problemen oder Nachteilen führen.
Die Einfärbung oder Aluminisierung von hochflexiblen Kunststoffolien ist in der Regel schwierig und bietet keinen zuverlässigen Lichtschutz, da infolge von Dehnung der Folie Risse in der Farbschicht oder in der Aluminiumbedampfungsschicht entstehen können, die partiell den Eintritt von Licht in die wirkstoffhaltige Polymermatrix ermöglichen und damit zum Abbau des Wirkstoffs in der Matrix führen können.

Als Alternative zu eingefärbten oder aluminiumbeschichteten Abdeckfolien bieten sich flexible, eingefärbte Gewebe an, die zuweilen hochelastisch sein können. Sie weisen jedoch den Nachteil auf, daß sie für eine mehrtägige Anwendung in der Regel nicht geeignet sind, weil sie den dabei auftretenden Umwelteinflüssen, insbesondere beim Duschen, Schwitzen, Saunabesuch etc., nicht standzuhalten vermögen.

Aluminisierte, lackierte oder eingefärbte Abdeckfolien haben zudem den Nachteil, daß sie optisch sehr auffallend sind und zu einer Stigmatisierung des Patienten führen können. Der Patient kann beim Tragen von TTS mit solchen Abdeckfolien als "kranke" Person erkennbar werden, was zu sozialen Ausgrenzungen und auf Seite des Patienten zu einer mangelnden Compliance oder Akzeptanz führen kann.

In JP 60069014 wird ein transdermales therapeutisches Sy-stem zur Behandlung von Dermatopathien beschrieben, das als Wirkstoff Psoralen enthält. Zur Aktivierung des Wirkstoffs ist eine UV-Bestrahlung erforderlich, so daß das Pflaster zwar im W-Bereich absorbierende Substanzen zum Schutz der darunterliegenden Hautpartien enthält, im für den Wirkstoff relevanten Wellenlängenbereich aber durchlässig ist.

Aufgabe der vorliegenden Erfindung war es deshalb, transdermal applizierbare Arzneistoffzubereitungen mit einem Gehalt an lichtempfindlichen Wirkstoffen bereitzustellen, bei welchen die Stabilität gegenüber Lichteinflüssen erhöht ist, ohne daß die vorstehend genannten Nachteile auftreten.

Erfindungsgemäß wird die Aufgabe dadurch gelöst, daß bei transdermalen therapeutischen Systemen (TTS), deren Aufbau eine wirkstoffhaltige Polymermatrix und eine Rückschicht umfaßt, und welche einen Gehalt an mindestens einem Wirkstoff aufweisen, der unter Einwirkung von W-Strahlung zersetzt wird und dadurch an Wirksamkeit verliert, mindestens eine im UV-Bereich absorbierende, farblose Substanz in der wirkstoffhaltigen Matrix homogen verteilt wird, z. B. in gelöster oder dispergierter Form, und/oder daß eine derartige Substanz in dessen Rückschicht (Abdeckfolie) homogen verteilt ist. Die im UV-Bereich absorbierende Substanz hat keine eigene pharmakologische Wirkung, d. h. sie ist selbst nicht therapeutisch wirksam und das Absorptionsmaximum der im UV-Bereich absorbierenden Substanz liegt innerhalb desjenigen Wellenlängenbereichs, durch welchen die Zersetzung des verwendeten Wirkstoffs verursacht wird.

Lichtempfindliche Wirkstoffe sind beispielsweise Nicotin, oder Wirkstoffe aus der Gruppe der Dihydropyridinderivate, z. B. Nifedipin oder Lacidipin, oder Gestagene, Vitamin B 12 und Antibiotika, sowie Salze solcher lichtempfindlicher Stoffe. Durch die Anwesenheit einer UV-absorbierenden, farblosen Substanz wird es ermöglicht, TTS herzustellen, die eine transparente Rückschicht und/oder eine transparente Wirkstoffmatrix aufweisen, und bei denen dennoch der Schutz der lichtempfindlichen Wirkstoffe vor lichtbedingter Zersetzung gewährleistet ist. Besonders vorteilhaft ist, daß auf diese Weise TTS hergestellt werden können, die vollkommen transparent sind und deshalb während des Tragens auf der Haut wenig auffallen. Dies ist insbesondere dann der Fall, wenn das TTS transparent und farblos ausgestaltet ist, wenn also sowohl die Rückschicht (Abdeckfolie) als auch die Polymermatrix, und gegebenenfalls weitere Schichten, transparent und farblos sind.

Als Materialien für die Abdeckfolie der erfindungsgemäßen TTS werden vorzugsweise transparente Folien aus Polyester, Polyethylen, Polypropylen, Polyurethan, Ethylen-Vinyl-Acetat, Polyethylenterephthalat (PET) oder Mischungen solcher Polymere verwendet.

Die wirkstoffhaltige Polymermatrix der erfindungsgemäßen TTS kann ein- oder mehrschichtig sein; vorzugsweise hat sie haftklebende Eigenschaften. Sie ist mit der Rückschicht (Abdeckfolie) fest verbunden bzw. bildet mit dieser ein Laminat. Die hautseitige, haftklebende Oberfläche der Polymermatrix wird üblicherweise von einer abziehbaren Schutzschicht oder Schutzfolie bedeckt, die vor der Applikation entfernt wird. Auch diese Schutzfolie kann lichtundurchlässig ausgestaltet sein.
Als Grundmaterialien für die Polymermatrix der erfindungsgemäßen TTS werden bevorzugt Polyacrylate, Polyisobutylene, Polydimethylsiloxane, Styrol-Isopren-Block-Copolymere oder Isoprenpolymere mit oder ohne synthetischen oder partialsynthetischen Polymeren verwendet.

In jedem Fall wird durch die Anwesenheit einer im UV-Bereich absorbierenden Substanz, auch UV-Absorber oder UV-Blocker genannt, bewirkt, daß der lichtempfindliche Wirkstoff vor photochemischer Zersetzung geschützt wird.
Unter UV-Bereich wird der Bereich des elektromagnetischen Spektrums verstanden, der zwischen 100 nm und 400 nm liegt. Für den vorgesehenen Zweck ist es in den meisten Fällen ausreichend, wenn die UV-absorbierende(n) Substanz(en) im Bereich von 250 nm bis 400 nm absorbieren. Bevorzugt werden solche UV-absorbierende Substanzen verwendet, die im UV-A-Bereich und/oder im UV-B-Bereich absorbieren (sogenannte UV-A-Absorber oder UV-B-Absorber).

Hinsichtlich der Auswahl des UV-Absorbers wird bevorzugt, daß dessen Absorptionsmaximum innerhalb desjenigen Wellenlängenbereichs liegt, durch welchen die Zersetzung des verwendeten Wirkstoffs verursacht wird.

Um einen Schutz vor photochemischer Zersetzung über einen breiteren UV-Spektralbereich zu erreichen, ist es vorteilhaft, wenn die erfindungsgemäßen TTS eine Kombination von mindestens zwei im UV-Bereich absorbierenden Substanzen enthalten, die unterschiedliche Absorptionsmaxima aufweisen.
Grundsätzlich werden bevorzugt solche UV-Absorber eingesetzt, deren Unbedenklichkeit bei der Verwendung in Kosmetikprodukten bereits nachgewiesen wurde, oder deren Anwendung auf der Haut toxikologisch unbedenklich ist.

Der gesamte Mengenanteil des/der zugesetzten UV-Absorber liegt vorzugsweise im Bereich von 1-20 Gew.-%, besonders bevorzugt im Bereich von 5-10 Gew.-%, jeweils bezogen auf ein TTS.

Die im UV-Bereich absorbierende(n) Substanz(en) wird/werden bevorzugt aus Gruppe ausgewählt, die p-Aminobenzoesäure und Aminobenzoesäurederivate, vorzugsweise 4-Dimethylaminobenzoesäure-2-ethyl-hexylester, 4-bis(polyethoxyl)aminobenzoesäure-polyethoxyethylester, sowie Zimtsäure und ihre Derivate, vorzugsweise 4-Methoxyzimtsäureisoamylester, 4-Methoxyzimtsäure-2-ethylhexylester, sowie 3-Benzylidenbornan-2-on und Benzylidenbornan-2-on-Derivate, vorzugsweise 3-(4')Methylbenzyliden-bornan-2-on, 3-(4-Sulfo)benzylidenbornan-2-on, 3-(4'-Trimethylaimnonium)benzylidenbornan2-on-Methylsulfat, sowie Salicylsäurederivate, vorzugsweise 4-Isopropylbenzylsalicylat, Salicylsäure-2-ethylhexylester, 3,3,5-Trimethyl-cyclohexyl-salicylat, sowie 2,4,6-Trianilin-p-(carbo-2'-ethyl-hexyl-1'-oxy)-1,3,5-triazin, 3-Imidazol-4-yl-acrylsäure und ihre Ester, 2-Phenylenbenzimidazol-5-sulfonsäure und ihre K-, Na- und Triethanolamin (=TEA)-Salze, 2-Cyan-3,3-diphenyl-acrylsäure, Terephthaloyliden-dicampher-sulfonsäure, Butylmethoxy-dibenzoyl-methan, sowie Benzophenone oder Benzophenon-Derivate, vorzugsweise Benzophenon-3, Benzophenon-4, umfaßt.

Die Erfindung und ihre vorteilhaften Eigenschaften werden anhand des folgenden Beispiels näher erläutert.

### Beispiel:

Es wurden zwei Formulierungen (A, B) eines lichtempfindlichen Wirkstoffs aus der Gruppe der Gestagene hergestellt, die sich in ihrer Zusammensetzung dadurch unterscheiden, daß die eine Formulierung (B) 10 Gew.-% eines UV-Absorbers enthielt, während die andere Formulierung (A) bei ansonsten gleicher Zusammensetzung keinen UV-Absorber enthielt. Beide haftklebenden wirkstoffhaltigen Laminate wurden mit einer transparenten Abdeckfolie aus PET ausgestattet, wodurch ein "TTS" erhalten wurde.

Die Zusammensetzung der Formulierung (B) ist wie folgt: (alle Angaben in Gew.-%)

| | |
|---|---|
| 2,0 % | Gestagen |
| 87,6 % | Acrylatpolymer |
| 0,4 % | Vernetzer |
| 10,0 % | Eusolex^{®} 6300 |

Eusolex^{®} 6300 (Fa. Merck, Darmstadt) ist ein öllöslicher UV-B-Absorber (3-(4-Methylenbenzyliden)-campher).

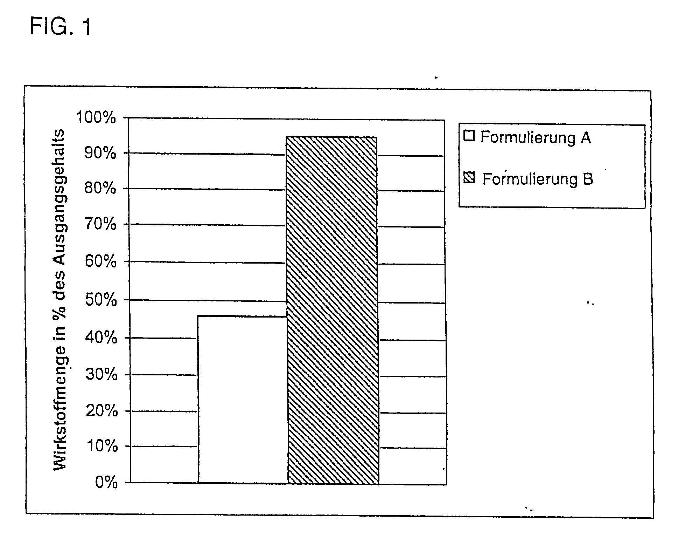

Zur Prüfung der Lichtschutzwirkung wurden beide mit PET-Folie bedeckten TTS-Formulierungen mit Xenon-Licht gemäß der ICH-Guideline "Note for guidance on the photostability testing of new active substances and medicinal products" (CPMP/ICH/279/95) bestrahlt. Die Bestrahlungszeit betrug 7 h, als Bestrahlungsquelle wurde eine Xenon-Lampe verwendet. Die verwendete Lichtquelle produziert konstruktionsbedingt eine Lichtabgabe vergleichbar mit dem D65/ID65-Emissionsstandard.
Anschließend wurde der Wirkstoffgehalt in den TTS bestimmt. Die Ergebnisse sind in FIG. 1 graphisch dargestellt.

Es zeigte sich, daß bei der TTS-Formulierung (B), welche UV-Absorber enthielt, ca. 95 % des eingesetzten lichtempfindlichen Wirkstoffs wiedergefunden werden konnte, während bei der TTS-Formulierung (A), die keinen UV-Absorber enthielt, nach der Bestrahlung lediglich 46 % der ursprünglich vorhandenen Wirkstoffmenge nachgewiesen werden konnten. Dies zeigt, daß der erfindungsgemäß vorgeschlagene Zusatz von UV-Absorbern die photochemische Zersetzung von Wirkstoffen verhindert und es somit ermöglicht, TTS mit einem Gehalt an lichtempfindlichen Wirkstoffen als transparente TTS herzustellen und damit deren Akzeptanz oder Compliance zu verbessern.

## Patentansprüche

1. Transdermale therapeutische Systeme (TTS), deren Aufbau eine wirkstoffhaltige Polymermatrix und eine Rückschicht umfaßt, mit einem Gehalt an mindestens einem wirkstoff, der unter Einwirkung von UV-Strahlung zersetzt wird und dadurch an Wirksamkeit verliert, **dadurch gekennzeichnet, daß** die genannten TTS mindestens eine im UV-Bereich absorbierende, farblose Substanz enthalten, die selbst nicht therapeutisch wirksam ist, und die in der Polymermatrix des TTS dispergiert oder gelöst ist und, und/oder die in der Rückschicht des TTS homogen verteilt ist, und daß das Absorgtionsmaximum der im UV-Bereich absorbierenden Substanz innerhalb desjenigen Wellenlängenbereichs liegt, durch welchen die zersetzung des verwendeten wirkstoffs verursacht wird, und wobei das TTS transparent und farblos ist.

2. TTS nach Anspruch 1, **dadurch gekennzeichnet, daß** sie mit einer transparenten Rückschicht ausgestattet sind, wobei als Rückschicht vorzugsweise eine transparente Folie aus Polyester, Polyethylen, Polypropylen, Polyurethan, Ethylen-Vinyl-Acetat oder Mischungen solcher Polymere verwendet wird.

3. TTS nach einem oder mehreren der vorangehenden Ansprüche, **dadurch gekennzeichnet, daß** die Polymermatrix im wesentlichen aus Polymeren hergestellt ist, die aus der Polyacrylate, Polyisobutylene, Polydimethylsiloxane, Styrol-Isopren-Block-Copolymere, Isoprenpolymere mit oder ohne synthetischen oder partialsynthetischen Polymeren umfassenden Gruppe ausgewählt sind.

4. TTS nach einem oder mehreren der vorangehenden Ansprüche, **dadurch gekennzeichnet, daß** sie die UV-absorbierende Substanz(en) in einem Anteil von 1-20 Gew.-% enthalten, vorzugsweise von 5-10 Gew.-%.

5. TTS nach einem oder mehreren der vorangehenden Ansprüche, **dadurch gekennzeichnet, daß** die im UV-Bereich absorbierende Substanz (en) im UV-A und/oder UV-B-Bereich absorbiert bzw. absorbieren, vorzugsweise im Wellenlängenbereich von 250-400 nm.

6. TTS nach einem oder mehreren der vorangehenden Ansprüche, **dadurch gekennzeichnet, daß** die im UV-Bereich absorbierende(n) Substanz (en) aus der Gruppe der nicht selbst therapeutisch wizksame(n) Substanzen ausgewählt ist/sind, die p-Aminobenzoesäure und Aminobenzoesäurederivate, vorzugsweise 4-Dimethylaminobenzoesäure-2-ethyl-hexylester, 4-bis(polyethoxyl)aminobenzoesäure-polyethoxyethylester, sowie Zimtsäure und ihre Derivate, vorzugsweise 4-Methoxyzimtsäureisoamylester, 4-Methoxyzimtsäure-2-ethylhexylester, sowie 3-Benzylidenbornan-2-on und Benzylidenbornan-2-on-Derivate, vorzugsweise 3-(4')Methylbenzyliden-bornan2-on, 3-(4-Sulfo)benzylidenbornan-2-on, 3-(4'-Trimethylammonium)benzylidenbornan-2-on-Methylsulfat, sowie Salicylsäurederivate, vorzugsweise 4-Isopropylbenzylsalicylat, Salicylsäure-2-ethylhexylester, 3,3,5-Trimethyl-cyclohexylsalicylat, sowie 2,4,6-Trianilin-p-(carbo-2'-ethyl-hexyl-1'-oxy)-1,3,5-triazin, 3-Imidazol-4-yl-acrylsäure und ihre Ester, 2-Phenylenbenzimidazol-5-sulfonsäure und ihre K-, Na- und Triethanolamin(=TEA)-Salze, 2-Cyan-3,3-diphenylacrylsäure, Terephthaloyliden-dicampher-aulfonsäure, Butylmethoxy-dibenzoyl-methan, sowie Benzophenone oder Benzophenon-Derivate, vorzugsweise Benzophenon-3, Benzophenon-4, umfaßt.

7. TTS nach einem oder mehreren der vorangehenden Ansprüche, **dadurch gekennzeichnet, daß** sie eine Kombination von mindestens zwei im UV-Bereich absorbierenden Substanzen enthalten, die unterschiedliche Absorptionsmaxima aufweisen.

## Claims

1. Transdermal therapeutic systems (TTS), with a structure comprising an active agent containing polymer matrix, and a backing layer, with a content of at least one active agent which is subject to decomposition by UV-radiation and thereby looses efficacy,
**characterized in that** the said TTS contain at least one colorless substance absorbing light in the UV-spectrum, and which has no therapeutic effect, and which is dispersed or dissolved in the polymer matrix of the TTS and, and/or which is homogenously distributed in the backing layer of the TTS, and wherein the maximum of absorption of said substance absorbing light in the UV-spectrum is within the wavelength range that causes decomposition of said active agent, and wherein the TTS is transparent and colorless.

2. TTS according to claim 1,
**characterized in that** the TTS are equipped with a transparent backing layer, wherein preferably the backing layer is a transparent film of polyester, polyethylene, polypropylene, polyurethane, ethylene vinyl acetate, or mixtures of such polymers.

3. TTS according to one or more of the preceding claims,
**characterized in that** the polymer matrix is essentially made of polymers selected from the group comprising polyacrylates, polyisobutylenes, polydimethyl siloxanes, styrene-isoprene block copolymers, isoprene polymers with or without synthetic or partially synthetic polymers.

4. TTS according to one or more of the preceding claims,
**characterized in that** they contain the UV-absorbing substance(s) in a portion of 1-20%-wt., preferably of 5-10%-wt.

5. TTS according to one or more of the preceding claims,
**characterized in that** the substance(s) which absorb light in the UV-spectrum in the UV-A and/or the UV-B spectrum, preferably in the wavelength range of 250-400 nm.

6. TTS according to one or more of the preceding claims,
**characterized in that** the substance(s) absorbing light in the UV-spectrum is/are selected from the group of therapeutically non-active substances comprising p-aminobenzoic acid and aminobenzoic acid derivatives, preferably 4-dimethylaminobenzoic acid-2-ethylhexyl ester, 4-bis(polyethoxyl) inobenzoic acid-polyethoxyethyl ester, as well as cinnamic acid and its derivatives, preferably 4-methoxycinnamic acid isoamyl ester, 4-methoxycinnamic acid-2-ethylhexyl ester, as well as 3-benzylidene bornan-2-one and benzylidene bornan-2-one derivatives, preferably 3-(4')methylbenzylidene-boman-2-one, 3-(4-sulfo)benzylidene bornan-2-on, 3-(4'-trimethylammonium)benzylidene boman-2-one-methyl sulphate, as well as salicylic acid derivatives, preferably 4-isopropylbenzyl salicylate, salicylic acid-2-ethylhexyl ester, 3,3,5-trimethyl-cyclohexyl salicylate, as well as 2,4,6-trianiline-p-(carbo-2'-ethyl-hexyl-1'-oxy)-1,3,5-triazine, 3-imidazol-4-yl-acrylic acid and corresponding esters, 2-phenylene benzimidazol-5-sulfonic acid and corresponding K-, Na- and triethanol amine (=TEA) salts, 2-cyano-3,3-diphenyl-acrylic acid, terephthaloylidene dicamphor sulfonic acid, butylmethoxydibenzoylmethane, as well as benzophenones or benzophenone derivatives, preferably benzophenone-3, benzophenone-4.

7. TTS according to one or more of the preceding claims,
**characterized in that** the TTS contain a combination of at least two substances absorbing light in the UV-spectrum with different absorption maxima.

## Revendications

1. Systèmes thérapeutiques transdermiques (STT) dont la structure comprend une matrice polymère contenant une substance active et une couche support, qui contient au moins un principe actif se décomposant sous l'action de la lumière et conséquemment perd de son activité, **caractérisés en ce que** ledit STT contient au moins une substance incolore qui absorbe dans la région des UV, ne présente pas en soi d'activité thérapeutique, est dispersé ou dissous dans la matrice polymère du STT, et/ou est répartie de manière homogène dans la couche support du STT et **en ce que** le maximum d'absorption de la substance qui absorbe dans la région des UV est compris dans la région des longueurs d'ondes qui provoque la décomposition du principe actif employé et le STT est ainsi transparent et incolore.

2. STT selon la revendication 1,
**caractérisés en ce qu'**ils sont pourvus d'une couche-support transparente, de sorte que pour la couche support soit de préférence employé film transparent en polyester, polyéthylène, polypropylène, polyuréthane, éthylène-acétate de vinyle ou de mélanges de ces polymères est utilisé comme couche support.

3. STT selon une ou plusieurs des revendications précédentes,
**caractérisés en ce que** la matrice polymère est essentiellement obtenue à partir de polymères qui sont choisis parmi les polyacrylates, les polyisobutylènes, les polydiméthylsiloxanes, les copolymères séquencés styrène-isoprène, les polymères d'isoprène avec ou sans groupe de polymères synthétiques ou partiellement synthétiques.

4. STT selon l'une ou plusieurs des revendications précédentes,
**caractérisés en ce qu'**ils contiennent la ou les substance(s) absorbant dans la région des UV en une proportion de 1 à 20% en poids, de préférence de 5 à 10% en poids.

5. STT selon l'une ou plusieurs des revendications précédentes,
**caractérisés en ce que** la ou les substance(s) absorbant dans la région des UV absorbe(nt) dans la région des UV-A et/ou dans la région des UV-B, et de préférence dans la gamme de longueurs d'onde de 250 à 400 nm.

6. STT selon l'une ou plusieurs des revendications précédentes,
**caractérisés en ce que** la ou les substance(s) absorbant dans la région des UV est/sont choisi(s) dans le groupe des substances n'ayant pas d'activité thérapeutique comprenant
- l'acide p-aminobenzoïque et les dérivés d'acide aminobenzoïque, de préférence l'ester 2-éthylhexylique de l'acide 4-diméthylaminobenzoïque, l'ester polyéthoxyéthylique du 4-bis-(polyéthoxyl)-aminobenzoique,
- ainsi que l'acide cinnamique et ses dérivés, par exemple l'ester isoamylique de l'acide 4-méthoxycinnamique, l'ester 2-éthylhexylique de l'acide 4-méthoxy-cinnamique,
- ainsi que le 3-benzylidènebornan-2-one et les dérivés de benzylidèneboman-2-one, de préférence le 3-(4')-méthylbenzylidène-bornan-2-one, le 3-(4-sulfo)-benzylidenboman-2-one, le sulfate méthylique du 3-(4'-triméthylammonium)-benzylidenboman-2-one,
- ainsi que les dérivés de l'acide salicylique, de préférence l'ester 2-éthylhexylique de l'acide 4-isopropylbenzyl salicylique, le 3,3,5-triméthylcyclohexyl salicylate
- ainsi que la 2,4,6- trianilino-p-(carbo-2'-éthylhexyl-1'-oxy) -1,3,5-triazine, l'acide 3-imidazol-4-yl-acrylique et ses esters, l'acide 2-phénylenbenzimidazol-5-sulfonique et ses sels de K, Na et triéthanolamine (=TEA), l'acide 2-cyano-3,3-diphénylacrylique, l'acide téréphtaloylide-dicamphre, le butylméthoxydibenzoylméthane,
- ainsi que la benzophénone et les dérivés de benzophénone, de préférence le benzophénone-3, le benzophénone-4.

7. STT selon l'une ou plusieurs des revendications précédentes,
**caractérisés en ce qu'**ils contiennent une combinaison d'au moins deux substances absorbant dans la région des UV qui présentent des maxima d'absorption différents.
